(19) Europäisches Patentamt
European Patent Office
Office européen des brevets

(11) Veröffentlichungsnummer: **0 005 821**
**B1**

(12) **EUROPÄISCHE PATENTSCHRIFT**

(45) Veröffentlichungstag der Patentschrift: **08.12.82**

(51) Int. Cl.³: **C 07 C 91/42,** C 07 C 93/14, C 07 C 103/38, A 61 K 31/13

(21) Anmeldenummer: **79101641.3**

(22) Anmeldetag: **29.05.79**

(54) **Indanaminderivate, Verfahren zur Herstellung derselben und Arzneimittel, welche diese enthalten.**

(30) Priorität: **30.05.78 JP 63821/78**
**30.05.78 JP 63822/78**
**26.03.79 JP 34349/79**

(43) Veröffentlichungstag der Anmeldung:
**12.12.79 Patentblatt 79/25**

(45) Bekanntmachung des Hinweises auf die Patenterteilung:
**08.12.82 Patentblatt 82/49**

(84) Benannte Vertragsstaaten:
**AT BE CH DE FR GB IT SE**

(56) Entgegenhaltungen:
**AT-B-293 371**
**DE-A-1 443 664**
**US-A-3 142 619**
**US-A-3 196 181**
**US-A-3 746 495**
**CHEMIKER ZEITUNG, 79. Jahrgang 1955, V. FRANZEN UND H. KRAUCH, »Die Curtius-Reaktion«, Seiten 772, 773.**

**Die Akte enthält technische Angaben, die nach dem Eingang der Anmeldung eingereicht wurden und die nicht in dieser Patentschrift enthalten sind.**

(73) Patentinhaber: **Hokuriku Pharmaceutical Co.,Ltd, 1-Chome, 3-14 Tatekawacho Katsuyamashi, Fukui (JP)**

(72) Erfinder: **Kato, Hideo, Dr., 1-11-27 Motomachi, Katsuyamashi Fukui (JP)**
Erfinder: **Koshinaka, Eiichi, 2-6-3 Asahicho, Katsuyamashi Fukui (JP)**
Erfinder: **Ogawa, Nobuo, 2-6-5 Asahicho, Katsuyamashi Fukui (JP)**
Erfinder: **Kurata, Sakae, 1-6-56 Asahicho, Katsuyamashi Fukui (JP)**
Erfinder: **Yamagishi, Kagari, 2-5-44 Asahicho, Katsuyamashi Fukui (JP)**

(74) Vertreter: **Werner, Hans-Karsten, Dr. et al, Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Indanaminderivate, Verfahren zur Herstellung derselben und Arzneimittel, welche diese enthalten

Diese Erfindung bezieht sich auf neue Indanaminderivate der Formel

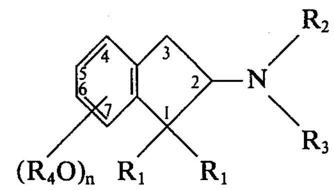

(I)

wobei $R_1$ eine niedere Alkylgruppe darstellt; $R_2$ und $R_3$ gleich oder verschieden sind und ein Wasserstoffatom, eine niedere Alkylgruppe oder eine Acylgruppe sein kann; $R_4$ ein Wasserstoffatom oder eine niedere Alkylgruppe; n null, 1 oder 2 ist und deren pharmakologisch verträglichen nicht toxischen Salze.

Die durch die Formel (I) dargestellten Verbindungen dieser Erfindung besitzen eine starke analgetische Aktivität mit einer sehr verminderten Nebenwirkung. Deswegen werden die neuen Verbindungen als stark wirksame analgetische Mittel angesehen.

Der Ausdruck »niedere Alkylgruppe«, der in der Beschreibung und in den Ansprüchen dieser Erfindung verwendet wird, bedeutet eine Alkylgruppe, welche eine geradkettige oder verzweigtkettige Kohlenstoffkette mit 1–6 Kohlenstoffatomen besitzt, wie z. B. die Methylgruppe, Äthylgruppe, Propylgruppe, Isopropylgruppe, n-Butylgruppe, Isobutylgruppe, sec.-Butylgruppe, tert.-Butylgruppe, Amylgruppe, Isoamylgruppe oder n-Hexangruppe.

Der Ausdruck »Acylgruppe« bedeutet eine Acylgruppe, welche eine geradkettige oder verzweigtkettige Kohlenstoffkette mit 1–6 Kohlenstoffatomen besitzt, wie z. B. Formylgruppe, Acetylgruppe, Propionylgruppe, Butyrylgruppe oder Isobutyrylgruppe.

Indanamin wird auch »2-Aminoindan« bezeichnet und ist in J. Pharm. Exp. Therapy Bd., 133, Seite 400 (1961) beschrieben. Dort wird eine analgetische Aktivität der Verbindung bei Untersuchung an Mäusen angegeben.

Die vorbekannte Verbindung konnte jedoch bisher nicht als wirksames analgetisches Mittel verwendet werden, weil sie eine starke Toxität und ungünstige Nebenwirkungen aufweist.

Bevorzugte Beispiele der Verbindungen dieser Erfindung sind:

1,1-Dimethyl-6-methoxyindanamin
N-Äthyl-1,1-dimethyl-6-methoxyindanamin
1,1-Dimethyl-2-indanamin
N-Äthyl-1,1-dimethyl-2-indanamin
1,1-Dimethyl-5-methoxy-2-indanamin
1,1-Dimethyl-7-methoxy-2-indanamin
1,1-Dimethyl-5,6-dimethoxy-2-indanamin
1,1-Diäthyl-6-methoxy-2-indanamin und
N-Äthyl-1,1-diäthyl-6-methoxy-2-indanamin

Als pharmakologisch zulässige nicht toxische Salze der Verbindung dieser Erfindung werden als Beispiel die Additionssalze einer Säure, wie Chlorwasserstoffsäure, Bromwasserstoffsäure, Schwefelsäure, Salpetersäure, Phosphorsäure und dgl. genannt.

Die erfindungsgemäßen Verbindungen werden aus den Verbindungen der Formeln (II) oder (II-1) als Ausgangsstoffe mit üblichen chemischen Methoden, wie z. B. katalytische Hydrierung, Alkylierung, Acylierung, Reduktion, Hydrolyse, Curtius-Abbau und dergleichen hergestellt.

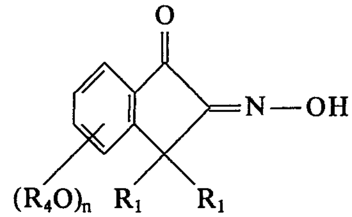

(II)

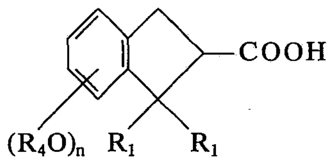

(II-1)

In dem nachstehend angegebenen chemischen Schema werden die verschiedenen Herstellungsverfahren veranschaulicht.

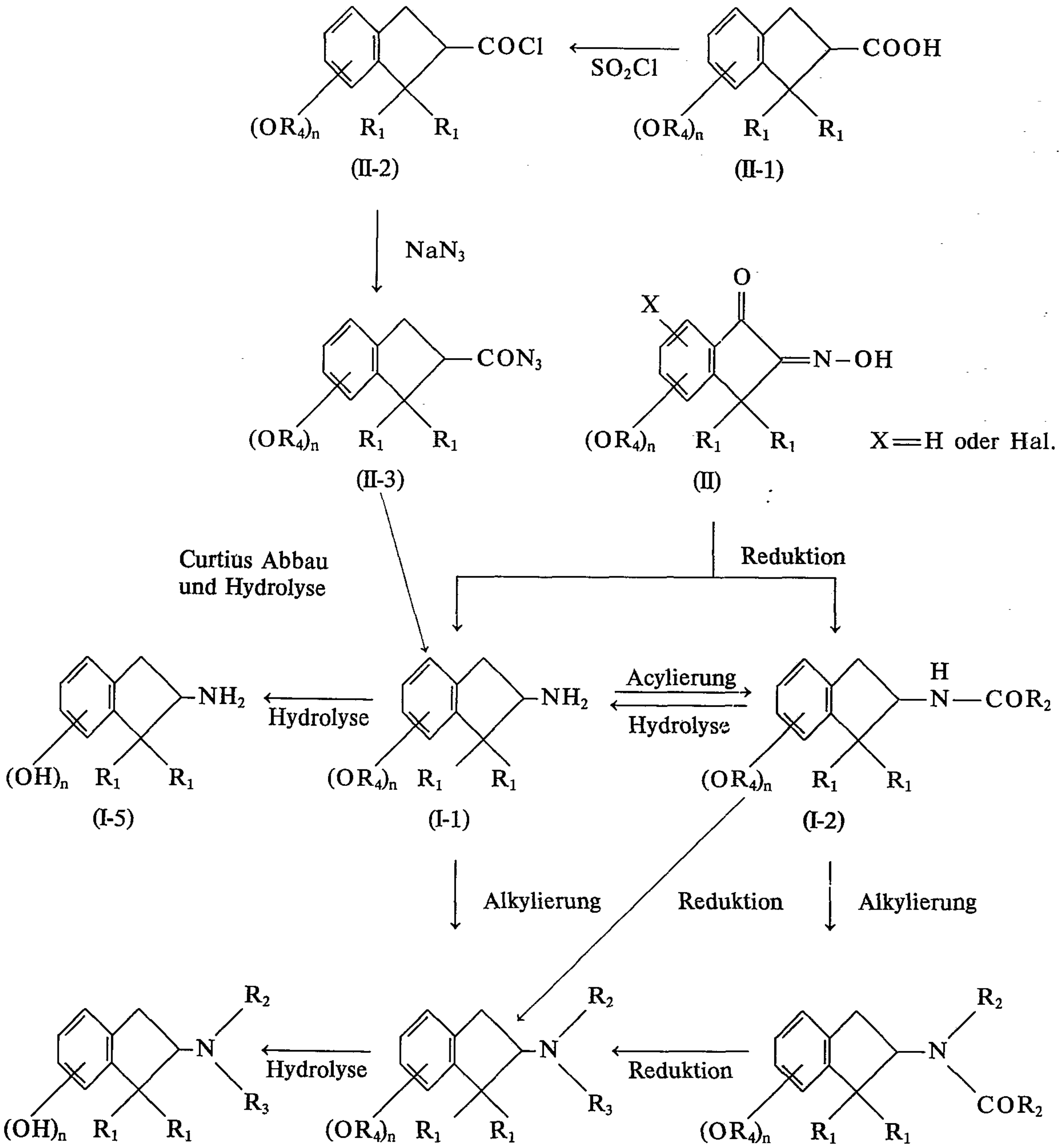


(II-2) (II-1) (II-3) (II) (I-5) (I-1) (I-2) (I-6) (I-3) (I-4)

Die Hydroxyliminoverbindung der Formel II wird in Gegenwart von Raney-Nickel, Pt-Oxid, Palladium-Kohle und dergleichen, vorzugsweise Palladium-Kohle, in einem organischen Lösungsmittel einschließlich einer aliphatischen Carbonsäure unter Druck und durch Erhitzen einer katalytischen Hydrierung unterworfen, damit die Verbindungen der Formeln (I-1) oder (I-2) erhalten werden können. Als organische Lösungsmittel kommen in Frage: Essigsäure, Äthanol, Methanol und dergleichen, vorzugsweise Essigsäure.

Die Hydrierung wird unter Druck zwischen 20 bis 50 atms., vorzugsweise 6 bis 30 atms., und durch Erhitzen auf eine Temperatur zwischen 50 bis 100°C, vorzugsweise 70 bis 80°C, während einer Reaktionsdauer von 2 bis 10 Stunden, vorzugsweise von 6 bis 7 Stunden, durchgeführt.

Die Verbindung der Formel (I-2) kann weiter durch Hydrolyse zu einer Verbindung der Formel (I-1) überführt werden. Die Hydrolyse wird in Gegenwart von NaOH oder ROH in einem organischen Lösungsmittel, wie z. B. Äthylenglykol, unter Rückfluß durch Erhitzen durchgeführt. Die Reaktionsdauer beträgt ungefähr 5 bis 48 Stunden, vorzugsweise 15 bis 24 Stunden.

Die Verbindungen der Formeln (I-2), (I-3) und (I-4) können aus der Verbindung (I-1) durch Monoalkylierung, Dialkylierung oder Acylierung hergestellt werden. Die Acylierungsprodukte der Formeln (I-2) und (I-4) können durch Reduktion zu den entsprechenden monoalkylierten oder dialkylierten Verbindungen der Formel (I-3) überführt werden.

Im einzelnen wird die Alkylierung durch Umsetzung der Verbindung der Formel (I-1) oder (I-2) mit Alkylhalogenid, wie z. B. Methylhalogenid, Äthylhalogenid, Propylhalogenid, Butylhalogenid, wobei Halogen ein Chloratom, Bromatom oder Jodidatom bedeutet, im äquimolaren Verhältnis oder im Überschuß bei einer Siedetemperatur des verwendeten Lösungsmittels während ungefähr 1 bis 20 Stunden, vorzugsweise 5 bis 10 Stunden, in Gegenwart von Alkalicarbonat, wie z. B. Soda oder Kaliumcarbonat, unter normalem Druck durchgeführt. Nötigenfalls können dabei Natriumhydrid, Natriumamid und dergleichen als Reaktionsbeschleuniger mitverwendet werden. Die Alkylierung kann ferner unter Verwendung von Ameisensäure und Carbonylverbindungen, wie z. B. Formaldehyd, Acetaldehyd und dergleichen in vergleichbaren Reaktionsbedingungen wie oben verwirklicht werden.

Die Acylierung einer Verbindung der Formel (I-1) mit einer aliphatischen Carbonsäure wie z. B. Ameisensäure, Essigsäure, Propionsäure, Buttersäure und dergleichen wird in der äquimolaren Menge oder im Überschuß der Säure bei einer Temperatur von 0° C bis Rückflußtemperatur während 0,5 bis ungefähr 6 Stunden, vorzugsweise 1 bis 1,5 Stunden, in Gegenwart von nicht polaren Lösungsmitteln, wie z. B. Benzol, Toluol, Xylol und dergleichen oder in Abwesenheit derselben unter normalem Druck durchgeführt.

Die so erhaltenen Acylierungsprodukte der Formeln (I-2) und (I-4) können durch milde Reduktion mit Hilfe des Lithiumaluminiumhydrid bei einer Rückflußtemperatur während ungefähr 3 bis 20 Stunden, vorzugsweise 4 bis 6 Stunden, in das entsprechende Alkylierungsprodukt der Formel (I-3) überführt werden. Die Reduktion soll wie üblich in Abwesenheit des Wassers durchgeführt werden. Der Curtiussche Abbau zur Herstellung der Verbindung der Formel (I-1) aus der Verbindung der Formel (II-3) wird durch Erhitzen im allgemeinen in Gegenwart von Säuren, wie z. B. Salzsäure, Schwefelsäure, Essigsäure und dergleichen oder Wasser, einzeln oder im Gemisch bei einer Rückflußtemperatur während 1 bis 10 Stunden, vorzugsweise 3 bis 5 Stunden, durchgeführt.

Das beim Curtiusschen Abbau zwischenzeitlich entstehende Isocyanat kann durch Hydrolyse zur Verbindung der Formel (I-1) überführt werden.

Die Verbindung der Formel (II-3) wird aus Indencarbonsäure der Formel (II-1) durch Umsetzen mit dem Thionylchlorid im Überschuß bei Raumtemperatur während 0,5 bis 2 Stunden zunächst zum Säurechlorid überführt und durch Reaktion des so erhaltenen Säurechlorides der Formel (II-2) mit dem Natriumazid in einer äquimolaren Menge hergestellt.

Die Verbindungen der Formeln (I-1-6), welche erfindungsgemäß hergestellt werden, können nach dem üblichen chemischen Arbeitsverfahren isoliert und gereinigt werden, durch Konzentration, Umkristallisation, Säulenchromatographie usw. und können mit einer üblichen Arbeitsweise in ihre entsprechenden pharmakologisch zulässigen nicht toxischen Salze überführt werden.

Die Verbindung der Formel (II) ist neu und wird, wie im nachstehenden Reaktionsschema erläutert, hergestellt.

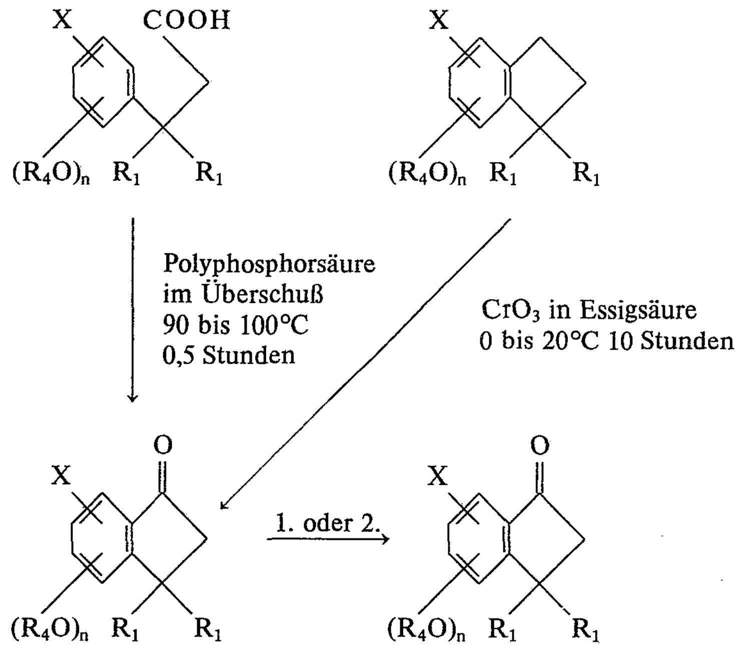


1. Isoamylnitrit, Salzsäure in Äthanol 50°C 2 bis 3 Stunden
2. $NaOCH_3$, Isoamylnitrit in Methanol 0 bis 5°C über Nacht

Die neuen Indanaminderivate der Formeln (I-1 bis 6) besitzen eine ausgezeichnete analgetische Wirkung und können als Arzneimittel und gleichzeitig als Zwischenprodukte zur Herstellung von Arzneimitteln verwendet werden.

Bei der klinischen Anwendung können die Verbindungen in einer Dosis von 10 mg bis 100 mg, bevorzugt 10 bis 30 mg, dreimal am Tag per os verabreicht oder als Injektion in einer entsprechenden Menge verwendet werden. Die Arzneimittel, welche die Verbindungen gemäß der Erfindung enthalten, können die üblichen Verdünnungsmittel und Träger enthalten.

Im folgenden wird die Erfindung anhand der Ausführungsbeispiele näher erläutert.

## Beispiel 1

### 1,1-Dimethyl-6-methoxy-2-indanamin-hydrochlorid

In 0,8 g 2,3-Dihydro-1,1-dimethyl-6-methoxy-1H-idene-2-carbonsäure wurden 2,0 ml Thionylchlorid unter Eiskühlung hinzugefügt. Das Gemisch wurde bei Raumtemperatur 0,5 Stunden lang gerührt, Dem Gemisch wurde getrocknetes Benzol zugefügt. um die überschüssige Menge von Thionylchlorid azeotropisch abzudestillieren. Das so erhaltene Carbonsäurechlorid wurde in 7 ml getrocknetem Benzol aufgelöst und 0,24 g Natriumazid wurden zugefügt. Das Gemisch wurde unter Rückfluß 22 Stunden lang erhitzt. Nach dem Abkühlen wurde das Gemisch filtriert. Dem Filtrat wurden 4 ml konzentrierte Salzsäure hinzugefügt und das Gemisch wurde unter Rückfluß 3 Stunden lang erhitzt. Nach Beendigung der Reaktion wurde die wässerige Phase mit einem Zusatz von Natriumhydroxid alkalisch gemacht und mit Äther extrahiert. Die ätherische Phase wurde mit Wasser gewaschen und dann getrocknet. Das Lösungsmittel wurde abdestilliert und der Rückstand wurde mit einem Zusatz von ätherischer Hydrochloridsäure in Hydrochlorid überführt. Nach Umkristallisierung aus Äthanol wurden 0,07 g farblose Prismen mit einem Schmelzpunkt von 238 bis 240° C (Zersetzung) erhalten.

Elementaranalyse für $C_{12}H_{17}NO \cdot H_2O \cdot HCl$

| | | | |
|---|---|---|---|
| Berechnet: | C 58,65 | H 8,20 | N 5,70 |
| Gefunden: | C 58,13 | H 8,43 | N 5,40 |

## Beispiel 2

### 1,1-Dimethyl-6-methoxy-2-indanamin-hydrochlorid

In 100 ml Essigsäure wurden 10 g 3,3-Dimethyl-2-hydroxylimino-5-methoxy-1-indanon aufgelöst. Nach Zusatz von 4 g 5%iger Palladium-Kohle wurde die Lösung unter Druck und durch Erhitzen (30 kg/cm², 80° C) 6,5 Stunden lang einer katalytischen Reduktion unterworfen. Der Katalysator wurde

abfiltriert und das Filtrat wurde unter vermindertem Druck getrocknet. Nach Zugabe von 10% wäßriger Salzsäure zum Rückstand wurde das Gemisch mit Äther gewaschen. Die wässerige Phase wurde mit Kaliumcarbonat alkalisch gemacht und mit Äther extrahiert. Die ätherische Phase wurde mit Wasser gewaschen und getrocknet. Nach Abdestillation des Lösungsmittels wurden 3,94 g hellgelbes Öl erhalten. Das Öl wurde mit einem Zusatz von äthanolischer Hydrochlorsäure in Hydrochlorid überführt. Nach Umkristallisierung aus Äthanol wurden farblose Prismen mit einem Schmelzpunkt von 238 bis 240° C (Zersetzung) erhalten. Das so erhaltene Hydrochlorid stellt ein gleiches IR Spektrum mit der im Beispiel 1 erhaltenen Verbindung auf und zeigt keine Senkung des Schmelzpunktes bei der Mischprobe.

## Beispiel 3

### 1,1-Dimethyl-6-äthoxy-2-indanamin-hydrochlorid

Zu einer Lösung von 30 g 3,3-Dimethyl-2-hydroxylimino-5-äthoxy-1-indanon in 150 ml Essigsäure wurden 12 g 10%ige Palladium-Kohle hinzugefügt. Das Gemisch wurde wie im Beispiel 2 angegeben behandelt um 12,3 g Indanamin-hydrochlorid zu erhalten. Nach Umkristallisierung aus Äthanol-Äther wurden farblose Nadeln mit einem Schmelzpunkt von 270 bis 272° C (Zersetzung) erhalten.

Elementaranalyse für $C_{13}H_{19}NO \cdot HCl \cdot 1/4H_2O$

| | | | |
|---|---|---|---|
| Berechnet: | C 63,40 | H 8,39 | N 5,69 |
| Gefunden: | C 63,44 | H 8,48 | N 5,58 |

## Beispiel 4

### 1,1-Diäthyl-6-methoxy-2-indanamin-hydrochlorid

Zu einer Lösung von 7,42 g 3,3-Diäthyl-2-hydroxylimino-5-methoxy-1-indanon in 100 ml Essigsäure wurden 2,69 g 10%iger Palladium-Kohle hinzugefügt. Nach Behandlung des so erhaltenen Gemisches, wie im Beispiel 2 angegeben, wurden 2,46 g Indanamin-hydrochlorid erhalten, aus dem nach Umkristallisierung aus Äthanol-isopropyläther farblose Nadeln mit einem Schmelzpunkt von 218 bis 22° C erhalten wurden.

Elementaranalyse für $C_{14}H_{21}NO \cdot HCl \cdot 1/4H_2O$

| | | | |
|---|---|---|---|
| Berechnet: | C 64,60 | H 8,71 | N 5,38 |
| Gefunden: | C 64,54 | H 8,84 | N 5,46 |

## Beispiel 5

### 6-Methoxy-1,1,N,N-tetramethyl-2-indanamin-hydrochlorid

Das Gemisch aus 6,0 g 6-Methoxy-1,1-dimethyl-2-indanamin und 9,3 ml 37%iger wässeriger Formaldehydlösung und 6,58 ml Ameisensäure wurde bei einer Temperatur von 80 bis 100° C 4 Stunden lang unter Rührung erhitzt. Nach Beendigung der Reaktion wurde zum Reaktionsgemisch Wasser hinzugefügt. Das Reaktionsgemisch wurde mit Natriumhydroxid alkalisch gemacht und mit Äther extrahiert. Die ätherische Phase wurde mit Wasser gewaschen, getrocknet und abdestilliert. Der Rückstand wurde mit einem Zusatz von ätherischer Hydrochlorsäure in Hydrochlorid überführt um 4,41 g farblose Kristalle zu erhalten. Nach Umkristallisierung aus Äthanol-isopropyläther wurden farblose Nadeln mit einem Schmelzpunkt von 215 bis 216° C (Zersetzung) erhalten.

Elementaranalyse für $C_{14}H_{21}NO \cdot HCl \cdot 1/4H_2O$

| | | | |
|---|---|---|---|
| Berechnet: | C 64,60 | H 8,62 | N 5,38 |
| Gefunden: | C 64,42 | H 8,71 | N 5,23 |

## Beispiel 6

### N-Acethyl-6-methoxy-1,1-dimethyl-2-indanamin

a) Eine Lösung von 10 g 3,3-Dimethyl-2-hydroxyimino-5-methoxy-1-indanon in 100 ml Essigsäure wurde in Gegenwart von 4 g 5%iger Palladium-Kohle unter Druck und durch Erhitzen (30 kg/cm², 80° C) 6,5 Stunden lang einer katalytischen Reduktion unterworfen. Der Katalysator wurde abfiltriert und das

Filtrat wurde getrocknet und destilliert. Nach Zugabe einer 10%igen wässerigen Hydrochlorsäure wurde der erhaltene Rückstand mit Äther extrahiert. Die ätherische Phase wurde mit Wasser gewaschen, getrocknet und abdestilliert um 4,86 g farblose Kristalle zu erhalten. Nach Umkristallisierung aus Isopropyläther wurden farblose Kristalle mit einem Schmelzpunkt von 99 bis 100° C erhalten.

Elementaranalyse für $C_{14}H_{19}NO_2$

| | | | |
|---|---|---|---|
| Berechnet: | C 72,07 | H 8,21 | N 6,00 |
| Gefunden: | C 71,92 | H 8,39 | N 5,85 |

b) Die Verbindung kann auch durch Acetylierung von 1,1-Dimethyl-6-methoxy-2-indanamin mit Essigsäureanhydrid erhalten werden.

## Beispiel 7

### N-Äthyl-6-methoxy-1,1-dimethyl-2-indanamin-hydrochlorid

Zu einer Suspension von 2,45 g Lithiumaluminium-hydrid in 10 ml getrocknetem Tetrahydrofuran wurde eine Lösung von 5 g N-Acetyl-6-methoxy-1,1-dimethyl-2-indanamin in 10 ml getrocknetem Tetrahydrofuran zugegeben. Das Gemisch wurde bei Raumtemperatur 20 Minuten lang gerührt und dann unter Rückfluß 1 Stunde lang erhitzt. Nach Beendigung der Reaktion wurden zum Reaktionsgemisch nacheinander wässeriger Äther und Wasser unter Eiskühlung zugegeben. Dann wurde das Gemisch abgesaugt und das Filtrat wurde unter vermindertem Druck getrocknet und destilliert. Nach Zugabe von 10% wässeriger Hydrochlorsäure zum Rückstand wurde das Gemisch mit Äther gewaschen. Die wässerige Phase wurde mit Natriumhydroxid alkalisch gemacht und mit Äther extrahiert. Die ätherische Phase wurde mit Wasser gewaschen, getrocknet und abdestilliert. Der Rückstand wurde mit einem Zusatz von äthanolischer Hydrochlorsäure in Hydrochlorid überführt um 4,01 g farblose Kristalle zu erhalten. Nach Umkristallisierung aus Äthanol-isopropyläther wurden farblose Nadeln mit einem Schmelzpunkt von 217 bis 218° C (Zersetzung) erhalten.

Elementaranalyse für $C_{14}H_{21}NO \cdot HCl$

| | | | |
|---|---|---|---|
| Berechnet: | C 65,48 | H 8,64 | N 5,45 |
| Gefunden: | C 65,43 | H 8,76 | N 5,42 |

## Beispiel 8

### N-Acetyl-6-methoxy-1,1,N-trimethyl-2-indanamin

Eine Suspension von 5 g N-Acetyl-6-methoxy-1,1-dimethyl-2-indanamin und 2,1 g 50% Natriumhydrid in 70 ml Dimethylformamid wurde bei einer Temperatur von 30 bis 50° C eine Stunde lang unter Rührung erhitzt. Zu der Suspension wurden 7,63 g Methyliodid zugegeben. Das Gemisch wurde 15 Minuten lang bei einer Temperatur von 30 bis 50° C gerührt und dann 1 Stunde lang bei Raumtemperatur. Nach Beendigung der Reaktion wurde dem Reaktionsgemisch Wasser zugegeben und mit Äther extrahiert. Die ätherische Phase wurde mit Wasser gewaschen und getrocknet. Das Lösungsmittel wurde abdestilliert und der Rückstand wurde aus n-Hexan kristallisiert um 5,2 g farblose Kristalle zu erhalten. Nach Umkristallisierung aus Isopropyläther wurden farblose Kristalle mit einem Schmelzpunkt von 97 bis 98° C erhalten.

Elementaranalyse für $C_{15}H_{21}NO_2$

| | | | |
|---|---|---|---|
| Berechnet: | C 72,84 | H 8,56 | N 5,66 |
| Gefunden: | C 72,68 | H 8,66 | N 5,61 |

## Beispiel 9

### N-Äthyl-6-hydroxy-1,1-dimethyl-2-indanamin-hydrobromid

Zu 1,92 g N-Äthyl-6-methoxy-1,1-dimethyl-2-indanamin-hydrochlorid wurden 30 ml 48%ige Hydrobromsäure zugegeben. Das Gemisch wurde bei einer Temperatur von 100 bis 120° C 3,5 Stunden lang unter Rührung erhitzt. Nach Beendigung der Reaktion und Zusatz des Äthanols wurde das Gemisch abgesaugt um 2,08 g hellbraunes Pulver zu erhalten. Nach Umkristallisierung aus wässerigem Äthanol wurden braune Prismen mit einem Schmelzpunkt von 298 bis 299° C erhalten.

Elementaranalyse für $C_{13}H_{19}NO \cdot HBr$

| | | | |
|---|---|---|---|
| Berechnet: | C 54,55 | H 7,04 | N 4,89 |
| Gefunden: | C 54,33 | H 7,19 | N 4,77 |

## Beispiel 10

### 6-Hydroxy-1,1,N,N-tetramethyl-2-indanamin-hydrobromid

Wie im Beispiel 9 angegeben, wurden aus 2,5 g 6-Methoxy-1,1,N,N-tetramethyl-2-indanamin 2,61 g Hydrobromid in Form von hellgrünen, schuppenförmigen Kristallen mit einem Schmelzpunkt von 229 bis 230° C erhalten.

Elementaranalyse für $C_{13}H_{19}NO \cdot HBr \cdot H_2O$

| | | | |
|---|---|---|---|
| Berechnet: | C 51,32 | H 7,29 | N 4,60 |
| Gefunden: | C 51,08 | H 7,30 | N 4,49 |

## Beispiel 11

### N-Acetyl-6-äthoxy-1,1-dimethyl-2-indanamin

Wie im Beispiel 6 angegeben wurden aus 30 g 3,3-Dimethyl-2-hydroxyimino-5-äthoxy-1-indanon unter Verwendung von 12 g 10%iger Palladium-Kohle 10,9 g farblose schuppenförmige Kristalle mit einem Schmelzpunkt von 137 bis 138° C erhalten.

Elementaranalyse für $C_{15}H_{21}NO_2$

| | | | |
|---|---|---|---|
| Berechnet: | C 72,84 | H 8,56 | N 5,66 |
| Gefunden: | C 72,79 | H 8,83 | N 5,60 |

## Beispiel 12

### N-Äthyl-6-äthoxy-1,1-dimethyl-2-indanamin-hydrochlorid

Unter Verwendung von 4,0 g N-Acetyl-6-äthoxy-1,1-dimethyl-2-indanamin wurden, wie im Beispiel 7 angegeben, 3,42 g N-Acetyl-6-äthoxy-1,1-dimethyl-2-indanaminhydrochlorid in Form von farblosen Nadeln mit einem Schmelzpunkt von 207 bis 208° C erhalten.

Elementaranalyse für $C_{15}H_{23}NO \cdot HCl$

| | | | |
|---|---|---|---|
| Berechnet: | C 66,77 | H 8,97 | N 5,19 |
| Gefunden: | C 66,54 | H 9,21 | N 4,98 |

## Beispiel 13

### 6-Äthoxy-1,1,N,N-tetramethyl-2-indanamin-hydrochlorid

Wie in Beispiel 5 angegeben, wurden aus 4,0 g 6-Äthoxy-1,1-dimethyl-2-indanamin 2,16 g des gewünschten Hydrochlorides in Form von farblosen Nadeln mit einem Schmelzpunkt von 219 bis 220° C erhalten.

## Beispiel 14

### N-Acetyl-1,1-diäthyl-6-methoxy-2-indanamin

Unter Verwendung von 7,42 g 3,3-Diäthyl-2-hydroxyimino-5-methoxy-1-indanon als Ausgangsverbindung wurden, wie im Beispiel 6 angegeben, 3,28 g farblose Nadeln mit einem Schmelzpunkt von 115 bis 116° C erhalten.

Elementaranalyse für $C_{16}H_{23}NO_2$

| | | | |
|---|---|---|---|
| Berechnet: | C 73,53 | H 8,87 | N 5,36 |
| Gefunden: | C 73,65 | H 9,08 | N 5,33 |

### Beispiel 15

1,1,N-Triäthyl-6-methoxy-2-indanamin-hydrochlorid

Wie in Beispiel 7 angegeben wurden unter Verwendung von 3,0 g N-Acetyl-1,1-diäthyl-6-methoxy-2-indanamin als Ausgangsstoff 1,18 g des gewünschten Hydrochlorides in Form von farblosen Nadeln mit einem Schmelzpunkt von 208 bis 210° C erhalten.

Elementaranalyse für $C_{16}H_{25}NO \cdot HCl \cdot 1/4 H_2O$

| | | | |
|---|---|---|---|
| Berechnet: | C 66,65 | H 9,26 | N 4,86 |
| Gefunden: | C 66,91 | H 9,44 | N 4,98 |

### Beispiel 16

1,1-Diäthyl-N,N-dimethyl-6-methoxy-2-indanamin-hydrochlorid

Wie im Beispiel 5 angegeben wurde aus 6-Methoxy-1,1-diäthyl-2-indanamin das gewünschte Hydrochlorid in Form von farblosen Nadeln mit einem Schmelzpunkt von 176 bis 178° C erhalten.

Elementaranalyse für $C_{16}H_{25}NO \cdot HCl \cdot 1/4 H_2O$

| | | | |
|---|---|---|---|
| Berechnet: | C 66,65 | H 9,26 | N 4,86 |
| Gefunden: | C 66,46 | H 9,42 | N 4,88 |

### Beispiel 17

N-Äthoxycarbonyl-1,1-dimethyl-6-methoxy-2-indanamin

Zu einer Lösung von 4,3 g 6-Methoxy-1,1-dimethyl-2-indanaminhydrochlorid in 50 ml Wasser wurden 30 ml Äther und 1,5 g Natriumhydroxid zugegeben und gerührt. Dann wurden 2,64 g Äthylchlorocarbonat dem Gemisch tropfenweise zugegeben. Das Gemisch wurde bei Raumtemperatur über Nacht gerührt. Nach Filtrierung wurde die ätherische Phase abgetrennt und mit verdünnter Hydrochlorsäure und Wasser gewaschen, getrocknet und abdestilliert. Der so erhaltene Rückstand wurde aus Benzol umkristallisiert um 3,7 g farblose Nadeln mit einem Schmelzpunkt von 153 bis 154° C zu erhalten.

Elementaranalyse für $C_{15}H_{21}NO_3 \cdot 3/4 H_2O$

| | | | |
|---|---|---|---|
| Berechnet: | C 65,08 | H 8,19 | N 5,06 |
| Gefunden: | C 64,92 | H 7,89 | N 5,15 |

### Beispiel 18

1,1,N-Trimethyl-6-methoxy-2-indanamin-hydrochlorid

Zu einer Suspension von 3,3 g Lithium-aluminiumhydrid in 40 ml getrocknetem Tetrahydrofuran wurde eine Lösung von 3,3 g N-Äthoxycarbonyl-1,1-dimethyl-6-methoxy-2-indanamin in 40 ml getrocknetem Tetrahydrofuran zugegeben. Das Gemisch wurde unter Rückfluß 7 Stunden lang erhitzt. Dann wurde es mit dem im Beispiel 7 angegebenen Verfahren behandelt um 2,46 g des gewünschten Hydrochlorides zu erhalten. Bei Umkristallisierung aus Äthanol wurden farblose Nadeln mit einem Schmelzpunkt von 288 bis 289° C erhalten.

Elementaranalyse für $C_{13}H_{19}NO \cdot HCl$

| | | | |
|---|---|---|---|
| Berechnet: | C 64,59 | H 8,34 | N 5,79 |
| Gefunden: | C 64,53 | H 8,62 | N 5,75 |

### Beispiel 19

1,1-Dimethyl-2-indanamin-hydrochlorid

Wie im Beispiel 2 angegeben, jedoch unter Verwendung von 22,3 g 3,3-Dimethyl-2-hydroxyimino-1-indanon wurden 8,4 g des gewünschten Hydrochlorid in Form von farblosen Nadeln mit einem Schmelzpunkt von 251 bis 252° C erhalten.

Elementaranalyse für $C_{11}H_{14}N \cdot HCl \cdot 1/2H_2O$

| | | | |
|---|---|---|---|
| Berechnet: | C 63,91 | H 8,29 | N 6,78 |
| Gefunden: | C 64,10 | H 8,10 | N 6,63 |

## Beispiel 20

### N-Acetyl-1,1-dimethyl-2-indanamin

Die im Beispiel 19 zwischenzeitlich erhaltene ätherische Phase, welche Hydrochlorsäure enthält, wurde mit Wasser gewaschen, getrocknet und unter vermindertem Druck abdestilliert um 4,32 g farblose viskose Flüssigkeit mit einem Siedepunkt von 157 bis 161° C (2 mm Hg) zu erhalten.

Maß Spektrum als $C_{13}H_{17}NO$
m/e: 203 (M+), 144 (base peak)

## Beispiel 21

### 1,1-Dimethyl-5-methoxy-2-indanamin-hydrochlorid

Wie im Beispiel 19 angegeben, jedoch unter Verwendung von 30 g 3,3-Dimethyl-2-hydroxy-6-methoxy-1-indanon wurde das gewünschte Indanaminhydrochlorid, welches nach Umkristallisierung aus Äthanol 5,0 g farblose Prismen mit einem Schmelzpunkt von 279 bis 281° C ergibt, erhalten.

Elementaranalyse für $C_{12}H_{17}NO \cdot HCl$

| | | | |
|---|---|---|---|
| Berechnet: | C 63,91 | H 8,29 | N 6,78 |
| Gefunden: | C 64,10 | H 8,10 | N 6,63 |

## Beispiel 22

### N-Acetyl-1,1-dimethyl-5-methoxy-2-indanamin

Die im Beispiel 21 zwischenzeitlich erhaltene Ätherphase, welche Salzsäure enthält, wurde mit Wasser gewaschen, getrocknet und abdestilliert um 4,65 g hellbraune viskose Flüssigkeit zu erhalten.
Massenspektrum als $C_{14}H_{19}NO_2$
m/e: 233 (M+), 92 (base peak)

## Beispiel 23

### 1,1-Dimethyl-5,6-dimethoxy-2-indanamin-hydrochlorid

Wie im Beispiel 19 angegeben, jedoch unter Verwendung von 9,5 g 3,3-Dimethyl-2-hydroxyimino-5,6-dimethoxy-1-indanon, wurde das gewünschte Indanaminhydrochlorid erhalten, aus welchem nach Umkristallisierung aus Äthanolisopropyläther 0,86 g farblose, blattförmige Kristalle mit einem Schmelzpunkt von 228 bis 230° C erhalten wurden.

Elementaranalyse für $C_{13}H_{19}NO_2 \cdot HCl$

| | | | |
|---|---|---|---|
| Berechnet: | C 60,58 | H 7,82 | N 5,43 |
| Gefunden: | C 60,34 | H 8,05 | N 5,62 |

## Beispiel 24

### N-Acetyl-1,1-dimethyl-5,6-dimethoxy-2-indanamin

Die im Beispiel 23 zwischenzeitlich erhaltene Ätherphase, welche Salzsäure enthält, wurde mit Wasser gewaschen, getrocknet und abdestilliert. Der so erhaltene Rückstand wurde aus Benzin-Hexan umkristallisiert um 4,27 g farblose Nadeln mit einem Schmelzpunkt von 145 bis 146° C zu erhalten.

Elementaranalyse für $C_{15}H_{21}NO_3$

| | | | |
|---|---|---|---|
| Berechnet: | C 68,42 | H 8,04 | N 5,32 |
| Gefunden: | C 68,29 | H 8,28 | N 5,21 |

Beispiel 25

1,1-Dimethyl-7-methoxy-2-indanamin-hydrochlorid

Wie in Beispiel 6 angegeben, jedoch unter Verwendung von 6,0 g 7-Chloro-3,3-dimethyl-2-hydroxy-imino-4-methoxy-1-indanon wurde das gewünschte Indanaminhydrochlorid erhalten, welches nach Umkristallisierung aus Isopropanol-isopropyläther 0,7 g farblose Nadeln mit einem Schmelzpunkt von 248 bis 249° C ergibt.

Elementaranalyse für $C_{12}H_{17}NO \cdot HCl$

| | | | |
|---|---|---|---|
| Berechnet: | C 63,29 | H 7,97 | N 6,15 |
| Gefunden: | C 63,11 | H 8,13 | N 6,04 |

Beispiel 26

N-Acetyl-1,1-dimethyl-4-methoxy-2-indanamin

Wie im Beispiel 6 angegeben, jedoch unter Verwendung von 6,0 g 3,3-Dimethyl-2-hydroxyimino-7-methoxy-1-indanon wurde das gewünschte N-Acetylindanamin erhalten, aus welchem nach Umkristallisierung aus Benzin 0,85 g farblose blattförmige Kristalle mit einem Schmelzpunkt von 140 bis 141° C erhalten wurden.

Elementaranalyse für $C_{14}H_{19}NO_2$

| | | | |
|---|---|---|---|
| Berechnet: | C 72,07 | H 8,21 | N 6,00 |
| Gefunden: | C 72,25 | H 8,49 | N 5,83 |

Beispiel 27

N-Äthyl-1,1-dimethyl-4-methoxy-2-indanamin-hydrochlorid

Wie im Beispiel 7 angegeben, jedoch unter Verwendung von 1,4 g N-Acetyl-1,1-dimethyl-4-meth-oxy-2-indanamin, wurde das gewünschte N-Äthylindanaminhydrochlorid erhalten, aus welchem nach Umkristallisierung aus Äthanoläther 0,8 g farblose, blattförmige Kristalle mit einem Schmelzpunkt von 300° C oder darüber erhalten wurden.

Elementaranalyse für $C_{14}H_{21}NO \cdot HCl$

| | | | |
|---|---|---|---|
| Berechnet: | C 65,74 | H 8,67 | N 5,48 |
| Gefunden: | C 65,39 | H 8,92 | N 5,40 |

Beispiel 28

N-Äthyl-1,1-dimethyl-2-indanamin-hydrochlorid

Wie im Beispiel 7 angegeben, jedoch unter Verwendung von 2,82 g N-Acetyl-1,1-dimethyl-2-indanamin wurden 1,8 g des gewünschten Hydrochlorides erhalten, aus welchem nach Umkristallisierung aus Äthanolisopropyläther farblose Nadeln mit einem Schmelzpunkt von 203 bis 204°C erhalten wurden.

Elementaranalyse für $C_{13}H_{19}N \cdot HCl$

| | | | |
|---|---|---|---|
| Berechnet: | C 69,16 | H 8,93 | N 6,20 |
| Gefunden: | C 69,38 | H 9,19 | N 6,12 |

Beispiel 29

N-Äthyl-1,1-dimethyl-5-methoxy-2-indanamin-hydrochlorid

Wie im Beispiel 7 angegeben, jedoch unter Verwendung von 4,29 g N-Acetyl-1,1-dimethyl-5-meth-oxy-2-indanamin wurde das gewünschte N-Äthyl-hydrochlorid hergestellt, aus welchem nach Umkristallisierung 1,28 g farblose Prismen mit einem Schmelzpunkt von 239 bis 240°C erhalten wurden.

Elementaranalyse für $C_{14}H_{21}NO \cdot HCl \cdot 1/4H_2O$

| | | | |
|---|---|---|---|
| Berechnet: | C 64,60 | H 8,71 | N 5,38 |
| Gefunden: | C 64,37 | H 8,79 | N 5,42 |

Beispiel 30

N-Äthyl-1,1-dimethyl-5,6-dimethoxy-2-indanamin-hydrochlorid

Wie im Beispiel 7 angegeben, jedoch unter Verwendung von 2,63 g N-Acetyl-1,1-dimethyl-5,6-dimethoxy-2-indanamin, wurden 1,59 g des gewünschten N-Äthylhydrochlorides hergestellt, aus welchem nach Umkristallisierung aus Äthanol farblose Prismen mit einem Schmelzpunkt von 289 bis 290° C erhalten wurden.

Elementaranalyse für $C_{15}H_{23}NO_2 \cdot HCl$

| | | | |
|---|---|---|---|
| Berechnet: | C 63,04 | H 8,46 | N 4,90 |
| Gefunden: | C 62,88 | H 8,57 | N 4,99 |

Beispiel 31

1,1,N,N-Tetramethyl-2-indanamin-hydrochlorid

Wie in Beispiel 5 angegeben, jedoch unter Verwendung von 3,26 g 1,1-Dimethyl-2-indanamin wurde das gewünschte N-Dimethylindanaminhydrochlorid erhalten, aus welchem nach Umkristallisierung aus Äthanol-isopropyläther 3,4 g farblose Nadeln mit einem Schmelzpunkt von 263 bis 264°C hergestellt wurden.

Elementaranalyse für $C_{13}H_{19}N \cdot HCl \cdot 1/2H_2O$

| | | | |
|---|---|---|---|
| Berechnet: | C 66,51 | H 9,02 | N 5,97 |
| Gefunden: | C 66,61 | H 8,92 | N 5,91 |

**Patentansprüche**

1. Indanaminderivate der Formel (I)

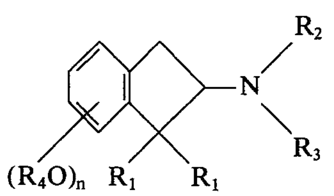

(I)

worin
$R_1$ eine niedere Alkylgruppe darstellt,
$R_2$ und $R_3$ gleich oder verschieden sind und ein Wasserstoffatom oder eine niedere Alkyl- bzw. Acylgruppe sein kann,
$R_4$ ein Wasserstoffatom oder eine niedere Alkylgruppe und
n null, 1 oder 2 ist
und deren pharmakologisch verträglichen nicht toxischen Salze.

2. Verfahren zur Herstellung der Verbindungen und ihrer Salze gemäß Anspruch 1, dadurch gekennzeichnet,

a) daß eine Verbindung der Formel (II)

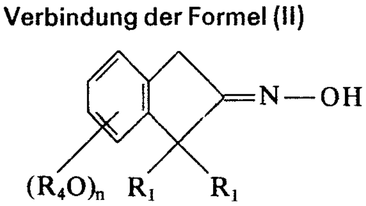

(II)

worin $R_1$ und $R_4$ die obengenannte Bedeutung haben, in Gegenwart eines organischen Lösungsmittels einschl. aliphatischer Carbonsäure katalytischer Hydrierung unterworfen wird oder

b) daß eine Verbindung der Formel (II-1)

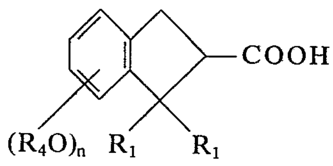

(II-1)

worin $R_1$ und $R_4$ die obengenannte Bedeutung haben über Carboxyazid dem Curtiusschen Abbau oder Hydrolyse unterworfen sind und die so erhaltene Verbindung nötigenfalls Hydrolyse, Acylierung, Alkylierung oder Reduktion unterworfen wird.

3. Analgetische Mittel, dadurch gekennzeichnet, daß sie als Wirkstoff mindestens eine Verbindung der Formel (I) oder ihre Salze enthalten.

**Claims**

1. Indanamine derivatives corresponding to the following formula

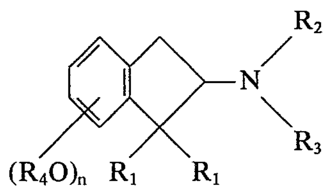

(I)

in which
$R_1$ represents a lower alkyl group,
$R_2$ and $R_3$ may be the same or different and represent a hydrogen atom or a lower alkyl or acyl group,
$R_4$ represents a hydrogen atom or a lower alkyl group and n = 0,1 or 2,
and their pharmacologically compatible non-toxic salts.

2. A process for producing the compounds claimed in claim 1 and their salts, characterised in that

a) a compound corresponding to the following Formula

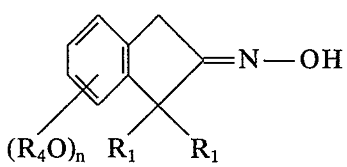

(II)

in which
$R_1$ and $R_4$ are as defined above,
is subjected to catalytic hydrogenation in the presence of an organic solvent, including aliphatic carboxylic acids, or

b) a compound corresponding to the following formula

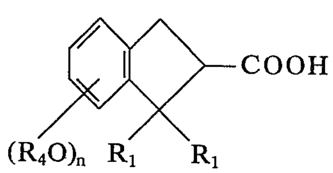

(II-1)

in which
$R_1$ and $R_4$ are as defined above,
is subjected through carboxyazide to Curtius degradation or to hydrolysis and the compound thus obtained is if necessary subjected to hydrolysis, acylation, alkylation or reduction.

3. Analgesics, characterised in that they contain at least one compound of the formula (I) or the salts thereof as the active ingredient.

**Revendications**

1. Dérivés d'hydrindamine de formule (I):

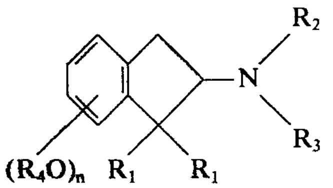

(I)

dans laquelle $R_1$ représente un radical alcoyle inférieur, $R_2$ et $R_3$ sont identiques ou différents et peuvent être un atome d'hydrogène, un radical alcoyle ou acyle inférieur, $R_4$ est un atome d'hydrogène ou un radical alcoyle inférieur et n est zéro, 1 ou 2, et leurs sels pharmacologiquement compatibles et non toxiques.

2. Procédé de fabrication des composés et de leurs sels selon la revendication 1, caractérisé en ce que:

a) un composé de formule (II):

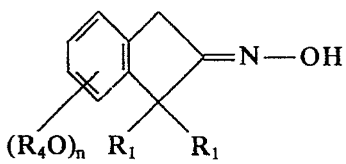

(II)

dans laquelle $R_1$ et $R_4$ ont les significations indiquées ci-dessus, est soumis à une hydrogénation catalytique en présence d'un solvant organique comprenant de l'acide carboxylique aliphatique, ou

b) un composé de formule (II-1):

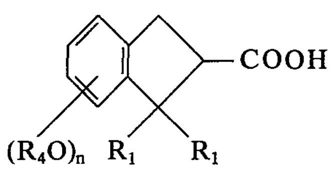

(II-1)

dans laquelle $R_1$ et $R_4$ ont les significations ci-dessus sont soumis à hydrolyse ou à la décomposition de Curtius au moyen d'azide carboxylique et en ce que le composé abtenu est en cas de besoin soumis à hydrolyse, acylation, alcoylation ou réduction.

3. Agents analgésiques, caractérisés en ce qu'ils comprennent en tant que substance active au moins un composé de formule (I) ou un de ses sels.